# EUROPEAN PATENT SPECIFICATION

(11) **EP 0 479 850 B1**
(45) Date of publication and mention of the grant of the patent: **01.05.1996**
(21) Application number: 90909782.6
(22) Date of filing: 12.06.1990
(51) Int. Cl.: A61K 31/045, C07C 31/20

(54) **PREPARATION FOR TOPICAL TREATMENT OF INFECTIONS CAUSED BY VIRUS, BACTERIA AND FUNGI**
ZUBEREITUNG FÜR DIE TOPISCHE BEHANDLUNG VON INFEKTIONEN DURCH VIREN, BAKTERIEN UND SCHIMMEL
PREPARATION DE TRAITEMENT LOCAL D'INFECTIONS PROVOQUEES PAR UN VIRUS, DES BACTERIES ET DES CHAMPIGNONS

(30) Priority: 13.06.1989 SE 8902124
(43) Date of publication of application: 15.04.1992
(73) Proprietor: FAERGEMANN, Jan, S-413 19 Göteborg (SE)
(72) Inventor: Swanbeck, Gunnar, S-436 00 Askim (SE); Faergemann,Jan, S-413 19 Göteborg (SE)
(74) Representative: Roth, Ernst Adolf Michael
(86) International application number: SE9000406
(87) International publication number: WO9015597

(56) References cited:
- US-A- 3 836 672
- US-A- 4 173 653
- Current Therapeutic Research, Vol. 43, No. 3, March 1988, J. FAERGEMANN: "The in vitro antimycotic activity of propane-1,2-diol,2-methyl-2,4-pentanediol, alclometasone dipropionate cream, and essex cream with the addition of propane-1,2-diol against Staphylococcus aureus, Staphylococcus epidermidis, Candida albicans, Trichophyton rubrum, and Pityrosporum orbiculare", pages 547-551.
- Sabouraudia, Vol. 18, No. 4, December 1980 J. FAERGEMANN et al.: "The antimycotic activity in vitro of five diols", see page 287-page 293
- Journal of Food Safety, Vol. 2, 1980 E.B. HERMAN et al.:"Antimicrobial action of short chain alcohols and glycols", see page 131 - page 139

## Description

### Background of the invention

The present invention refers to the use of pentane diol or hexane diol for the preparation of a composition for topical treatment of infections caused by herpesvirus, said diol being the active substance in said composition.

Diols or glycols are used as solvents, as anti-freezing agents or as vehicles in pharmaceutical preparations and some of them have an antimicrobial effect. A laboratory study of certain diols showed that the antimycotic activity was increased with an increasing length of the carbon chain.

So far propane-1,2-diol is the only diol widely used in dermatology. It is used in the treatment of Pityriasis versicolor, Pityrosporum folliculitis and Seborrhoeic dermatitis. It has even been active against Influenza A virus in vitro.

Certain laboratory tests indicate that pentane-1-5-diol has a higher activity against both fungi and bacteria than propane-1,2-diol (Faergemann et al: The antimycotic activity of five diols. Saubouradia 18:287-293, 1980). Nothing is however known about its effect in clinical treatment of infections caused by fungi and bacteria and nothing is known about its activity against virus, as far as we know. It further has a low oral toxicity and is non-irritating to the skin.

There is today no effective topical treatment available for the treatment of recurrent herpes labialis. Acyclovir given orally is effective but no effective topical formulation has been found so far.

### Summary of the invention

The object of the present invention is to provide a preparation that is effective in topical treatment of infections caused by virus, bacteria and fungi; especially herpesvirus and Staphylococcus aureus and Staphylococcus epidermidis. This has been provided by using a composition containing pentane diol or hexane diol. Pentane-1,5-diol is a preferred diol.

### Description of the invention

Study 1: In the study described below we found that pentane-1,5-diol is effective in the treatment of recurrent herpes labialis.

In vivo experiments were performed on 17 patients, 15 females and 2 males (22-40 years old), culture positive for Herpes simplex virus Type I and with recurrent Herpes simplex virus infection. The patients had at least three recurrent infections per year.

Pentane-1,5-diol was diluted in ethanol to give a clear solution containing 50% pentane-1,5-diol and 50% ethanol. This solution was applied five times daily for 5 days starting as early as possible when the first small vesicles appeared. The lesions were assessed using a 0-3 scale where 0 means healed and 3 severe lesions. The parameters studied were: erythema, vesicles, ulcer and crusts. The patients were studied as early as possible after start of lesions and then after 5 days of treatment.

Result: Fifteen out of the 17 patients has one or several recurrent herpes labialis infections during a 4-6 month period. In 13 of the 15 patients only minor changes were seen at day 5, such as e.g. a slight erythema or a little crust. All of these patients claimed that the episode was milder than usual and that the pain often disappeared already on the first treatment day. They found the preparation effective, easy to use and no side effects were seen.

In the last two patients the treatment first started when larger vesicles had appeared and they experienced none or only a slight reduction in their lesions compared to previous infections.

Being effective against herpes labialis it is likely that pentane-1,5-diol is effective also against herpes genitalis. In vitro experiments were also performed by testing the effect of pentane-1,5-diol against fibroblast cells infected with Herpes simplex virus type I. Pentane-1,5-diol was cytotoxic against the fibroblasts used to culture Herpes simplex virus type I in a dilution of 1/100. In higher dilutions no effects were seen.

Thus its activity in vitro against Herpes simplex type I virus could not be evaluated because it was cytotoxic to the cells used to culture the herpes virus in as high dilution as 1/100.

Pentane-1,5-diol has a low oral toxicity; LD₅₀ for rats is 5.89 g/kg and LD₅₀ for rabbits is greater than 20 ml/kg. It is essentially non-irritating to the skin and only very mildly irritating to the eyes.

Study 2: In this study the activity of propane-1,2-diol and pentane-1,5-diol against certain bacteria were compared. The activity is given in Table 1 as MIC (%), i.e. the minimum concentration that gave a total inhibition of the growth of the bacteria. It is seen from Table 1 that pentane-1,5-diol had the highest activity against all of the bacteria tested.

**Table 1**

| Bacteria | MIC (%) | |
|---|---|---|
| | Propane-1,2-diol | Pentane-1,5-diol |
| S. aureus | 20 | 5 |
| S. epidermidis | 20 | 5 |
| C. albicans | 10 | 4 |
| T. rubrum | 10 | 1 |
| P. ovale | 10 | 2 |

The above described studies refer to the use of pentane-1,5-diol, but it is likely that other pentane diols, hexane diol etc. have a corresponding effect. It is also likely that diols with longer carbon chains have a higher activity, but on the other hand they also have a higher toxicity. It is further likely that the diols according to the present invention have activity against infections caused by a variety of virus, bacteria and fungi. The concentration of the diol can vary from a high dilution to concentrated form.

## Claims

1. Use of pentane diol or hexane diol for the preparation of a composition for topical treatment of infections caused by herpesvirus, said diol being the active substance in said composition.

2. Use according to claim 1,
**characterized in**,
that the diol is pentane-1,5-diol.

3. Use according to claim 1 or 2,
**characterized in**,
that the composition contains a solvent for the diol, such as water, ether or ethanol.

## Patentansprüche

1. Verwendung von Pentandiol oder Hexandiol zur Herstellung einer Zusammensetzung zur topischen Behandlung von Infektionen durch Herpes-Viren, wobei das Diol die aktive Substanz in der Zusammensetzung ist.

2. Verwendung nach Anspruch 1, dadurch
**gekennzeichnet, daß**
das Diol Pentan-1,5-Diol ist.

3. Verwendung nach Anspruch 1 oder 2, dadurch
**gekennzeichnet, daß**
die Zusammensetzung ein Lösungsmittel für das Diol enthält, wie z.B. Wasser, Ether oder Ethanol.

## Revendications

1. Utilisation d'un pentane diol ou d'un hexane diol pour la préparation d'un composé destiné au traitement local d'infections provoquées par le virus de l'herpès, ledit diol constituant le principe actif dudit composé.

2. Utilisation selon la revendication 1, caractérisée en ce que le diol est un pentane-1,5-diol.

3. Utilisation selon la revendication 1 ou 2, caractérisée en ce que le composé contient un solvant pour le diol, tel que l'eau, l'éther ou l'éthanol.
